Europäisches Patentamt

European Patent Office

Office européen des brevets

(19)

(11) Numéro de publication: **0 274 363 B1**

(12) **FASCICULE DE BREVET EUROPEEN**

(45) Date de publication de fascicule du brevet: **29.04.92**   (51) Int. Cl.⁵: **A61K 7/48**

(21) Numéro de dépôt: **87830435.1**

(22) Date de dépôt: **14.12.87**

(54) **Produit et procédé pour un traitement hydratant efficace de la peau, utile notamment dans le domaine cosmétique.**

(30) Priorité: **19.12.86 IT 2279586**

(43) Date de publication de la demande:
**13.07.88 Bulletin 88/28**

(45) Mention de la délivrance du brevet:
**29.04.92 Bulletin 92/18**

(84) Etats contractants désignés:
**AT BE CH DE ES FR GB GR LI LU NL SE**

(56) Documents cités:
**AT-B- 300 199**
**FR-A- 2 473 887**
**FR-A- 2 540 381**
**GB-A- 2 013 609**
**US-A- 3 957 971**

(73) Titulaire: **Cosmetici Sorgente Panna S.p.A.**
**Via Castelvetro, 17/23**
**I-20154 Milano(IT)**

(72) Inventeur: **Curri Bertini, Sergio**
**Via Cagliero, 17**
**I-20125 Milan(IT)**

(74) Mandataire: **Vannini, Torquato et al**
**JACOBACCI-CASETTA & PERANI S.p.A. 7 Via**
**Visconti di Modrone**
**I-20122 Milan(IT)**

**Description**

La présente invention concerne un procédé pour effectuer un traitement hydratant efficace de la Peau: utilisable en Particulier: mais non exclusivement, dans le domaine cosmétique.

On sait que depuis toujours une partie importante de la recherche cosmétologique a pour objet principal de découvrir des substances, Préparations, formulations et procédés qui puissent etre utilisés de manière efficace et satisfaisante pour hydrater la peau.

Ainsi, par exemple, dans le brevet AT 300199 on décrit un procédé pour la préparation d'une poudre soluble en eau qui conserve inaltérés les effets physiologiques caractéristiques d'une eau minérale de source, par exemple d'une station thérmale.

Cette poudre est obtenue par un procédé grace auquel l'eau minérale naturelle est additionnée avec une substance choisie préférablement dans le groupe comprenant les eccipients et les colloides protecteurs et donc est séchée par pulvérisation.

Dans la demande de brevet FR 2473887 on décrit, au contraire, l'emploie des précurseurs des glucosaminoglycannes (GAG) comme par exemple l'acide hyalobiuronique ou l'acide N-acétylhyalobiuronique, pour le traitement de l'acrosyndrome vasculaire d'origine fonctionnelle et organique due à une insuffisante irroration sanguine de la peau.

Les précurseurs des glucosaminoglycannes sont administrés comme préparations sur la forme de solutions ou émulsions avec diluents inertes connus. Préférablement on utilise des émulsions huile dans l'eau, solutions aqueuses et hydroalcooliques.

On sait de meme que les liposomes (microdispersions de phospholipides dans l'eau) sont apparus depuis quelque temps dans le quadre de la recherce cosmétologique à cause de leur structure lamellaire (uni- ou plurilamellaire) semblable à celle des membranes cellulaires et donc en mesure de traverser la barrière épidermique, et à cause de la capacité de retenir de l'eau et donc de la transporter à travers les différentes couches de la peau à hydrater.

L'utilisation de liposomes comme vecteurs d'eau ou tout simplement comme porteurs de substances, principes actifs et analogues, retenus ou dissous - dans l'eau interlamellaire ou du compartiment central des liposomes eux-memes, a été largement suggérée et également confirmée expérimentalement.

Toutefois, au niveau de l'hydration pure et simple de la peau, l'éfficacité des techniques étudiées et essayées jusqu'à présent n'est pas révélée totalement satisfaisante. Par exemple, l'effet émollient reconnu d'un traitement à base de liposomes est toujours d'une durée très courte, probablement à cause du fait qu'il est du au seul apport d'eau exogène aux cellules les plus proches de la surface et qui, comme telle, s'évapore rapidement.

Pour obtenir une pénétration accrue dans les couches de la peau et un effet hydratant accru, on a suggéré et essayé depuis longtemps l'utilisation d'additifs humectants, par exemple glycérine, urée, sorbitol, pyroglutamate, etc. Mais si d'une part on peut dire que l'effet hydratant recheché est amélioré et satisfaisant, par contre l'incorporation de ces substances et/ou additifs dans des liposomes implique toute une série d'opérations qui ne sont pas d'exécution facile et qui genent à un degré parfois décourageant, d'un point de vue tant technique, qu'économique, la préparation de ces liposomes à l'échelle industrielle.

Le problème qui est à la base de la présente invention est de mettre à disposition un procédé pour un traitement hydratant efficace de la peau en mesure de surmonter les inconvénients exposés ci-dessus en référence à la technique connue, c'est-à-dire un procédé en mesure de garantir une hydratation de la peau plus que satisfaisante, non limitée seulement aux couches superficielles de celle-ci et n'impliquant pas de techniques particulières et difficiles pour la préparation à l'échelle industrielle et la conservation des liposomes respectifs.

Ce problème est résolu selon l'invention par un procédé pour le traitement hydratant de la peau, en particulier pour l'application cosmétologique, caractérisé en ce qu'il comprend une administration locale à la peau de liposomes qui contiennent des précurseurs biochimiques immédiats des glucosaminoglycannes désulfurés et eau minérale.

Selon un aspect de l'invention, les liposomes utilisés pour l'administration ci-dessus sont préparés (par des techniques classiques) dans l'eau minérale, en particulier une eau oligominérale.

Selon une autre caractéristique de l'invention, l'administration d'eau minérale par voie liposomique est effectuée par distribution de cette eau à l'état nébulisé ou atomisé à partir de petites bombes dans lesquelles l'eau minérale liposomique et un agent propulseur approprié ont été préalablement conditionnés.

L'invention se refère aussi à un produit pour un traitement hydratant efficace de la peau, utilisé en particulier, mais non exclusivement, dans le domaine cosmétique, caracterisé en ce qu'il comprend des liposomes qui contiennent eau oligominérale et précurseurs biochimiques immédiats des glucosaminoglycannes désulfurés.

2

EP 0 274 363 B1

D'autres caractéristiques et avantages de l'invention résulterons principalement de la description qui va suivre d'un exemple de mise en ouvre du procédé de l'invention, dont les résultats sont comparés à ceux de la tecnique connue, et en référence aux dessins annéxés, dans lesquels:

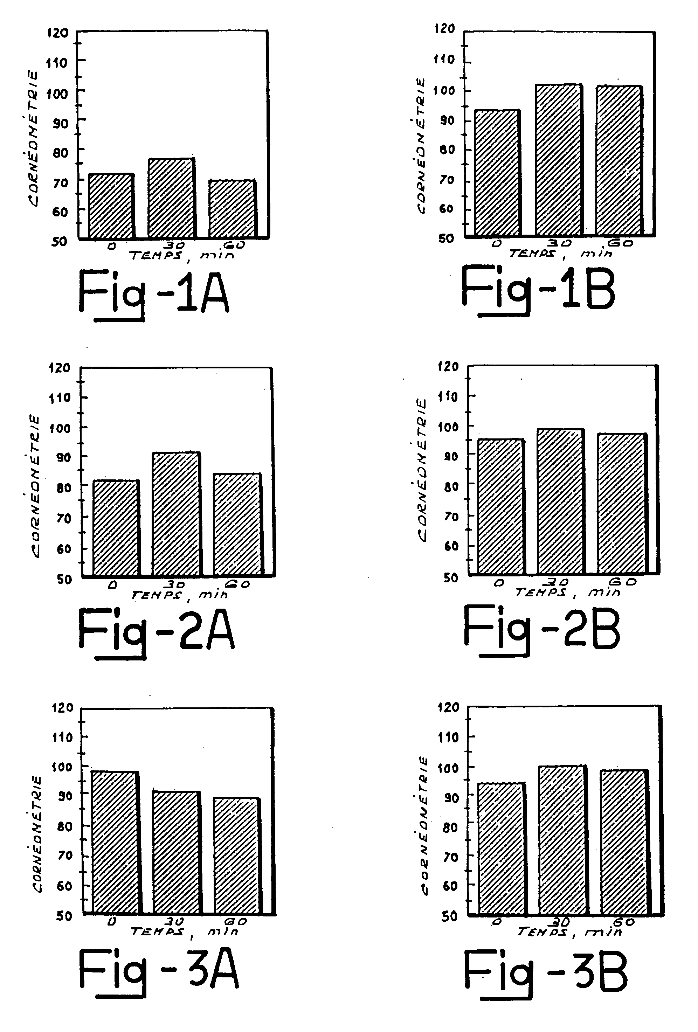

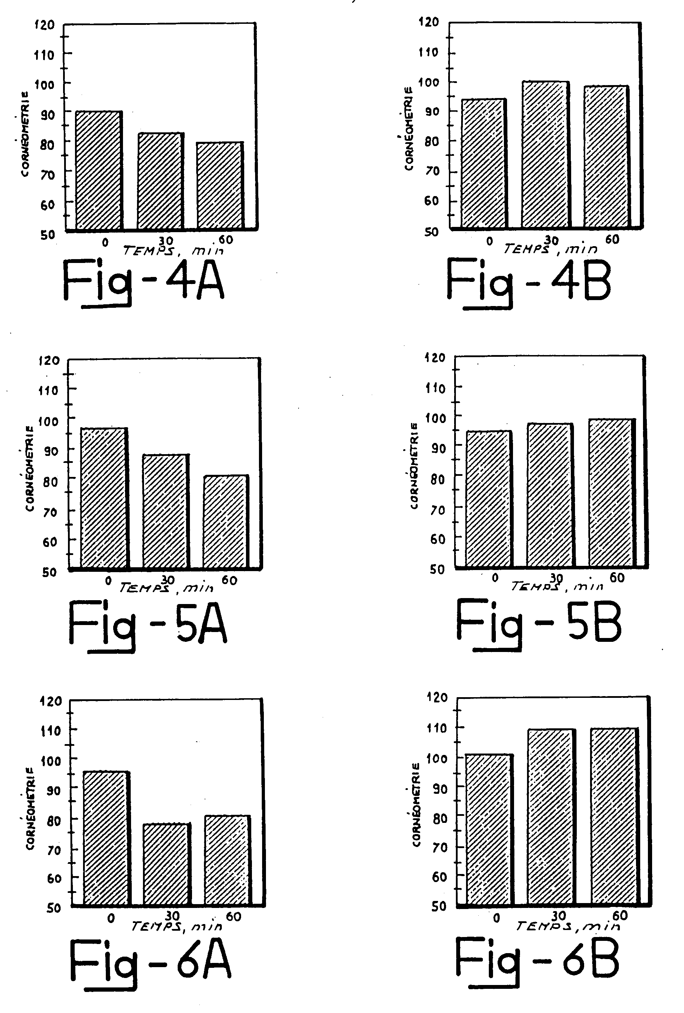

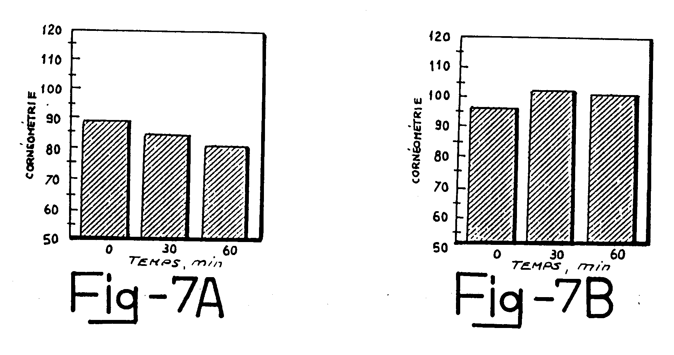

- les figures 1A à 7A représent des histogrammes relatifs à des données expérimentales relevées sur le visage de sujets soumis à un traitement par des liposomes de la technique classique et correspondant au front (figure 1A), aux régions temporo-zygomatiques droite (figure 2A) et gauche (figure 3A), aux joues droite (figure 4A) et gauche (figure 5A), au menton (figure 6A) et à l'hydratation moyenne du visage (figure 7A), respectivement, et
- les figures 1B à 7B représentent des histogrammes analogues relatifs à des données expérimentales relevées sur des sujets soumis à un traitement d'hydratation selon l'invention.

Les exemples suivants illustrent l'invention.

Exemple 1

Dans 100 ml d'eau oligominérale du commerce vendue sous le nom "Sorgente Panna", après traitement selon Deamer et collaborateurs (1976), on met sous forme microdispersée par traitement par les ultrasons 2 g De phosphatidylcholine purifiée, en ajoutant 500 mg de précurseurs biochimiques immédiats des glucosaminoglycannes désulfurés (GAG). Après agitation prolongée pendant 20 min, on laisse décanter pendant 24 h et on filtre ensuite le substrat pour obtenir une microdispersion opalescente à laquelle on ajoute en quantités appropriées des conservateurs de type classique.

Les liposomes d'eau oligominérale "Sorgente Panna" ainsi obtenus sont ensuite conditionnés dans de petites bombes du type spray en utilisant l'azote comme agent propulseur préféré.

Le produit ainsi conditionné est ensuite envoyé au stockage.

Exemple 2

Avec le même mode opératoire que celui décrit pour la préparation de liposomes d'eau oligominérale, on prépare des liposomes d'eau déminéralisée en partant des mêmes phospholipides (phosphatidylcholine), mais en utilisant au lieu des précurseurs GAG une solution de collagène à 10 %, dont on a largement décrit dans la littérature cosmétologique les propriétés d'hydratation de la couche cornée.

Dans ce cas également, les liposomes d'eau déminéralisée ainsi obtenus sont conditionnés dans de petites bombes spray avec un agent propulseur choisi parmi les gaz inertes, en particulier l'azote. Les bombes sont ensuite envoyées au stockage.

On détermine sur 10 sujets de sexe féminin âgés de 30 à 58 ans le gradient d'hydratation de la couche cornée en utilisant l'appareil CORNEOMETER CM 420 selon Schrader. Les déterminations sont effectuées dans les conditions initiales et 30 et 60 min après l'application de la préparation liposomale (liposomes d'eau déminéralisée et de collagène) obtenue à l'exemple 2, sur la peau du front, des régions temporo-zygomatiques droite et gauche, des joues droite et gauche et du menton.

Sur un second groupe de 20 sujets de sexe féminin de 18 à 42 ans, on détermine les valeurs cornéométriques dans les mêmes régions du visage aux mêmes temps qu'à l'expérience précédente, en effectuant le traitement avec des liposomes d'eau oligominérale "Sorgente Panna" obtenus selon l'exemple 1.

On soumet les données à l'analyse statistique, en calculant les moyennes et l'écart-type $\sigma$ et la significativité des différences selon le "T" de Student et l'analyse de la variante.

Les résultats des expériences faites sur les sujets du premier groupe (technique antérieure) sont rapportés dans le tableau I ci-après.

Les résultats de l'expérience sur les sujets du second groupe traités par les liposomes d'eau oligominérale "Sorgente Panna" plus les précurseurs de GAG (invention) sont indiqués dans le tableau II suivant.

Les données exposées dans les tableaux I et II sont résumées graphiquement dans les histogrammes des figures 1A à 7A et 1B à 7B, respectivement.

Il ressort de l'examen des données rapportées dans les tableaux et les figures que, tandis que la préparation de l'exemple 2 s'est révélée inefficace à 60 min pour augmenter les valeurs moyennes de l'hydratation cutanée, à l'exception des faibles augmentations à la trentième minute, dans toutes les régions cutanées du visage examinées, par contre, la préparation selon l'invention s'est révélée avoir un effet hydratant déjà évident à la trentième minute et se maintenant dans la plupart des cas jusqu'à la soixantième minute, c'est-à-dire dans t"ute la durée de l'expérience.

3

L'allure des valeurs moyennes globales se rapportant à la somme des valeurs cornéométriques de toutes les régions cutanées du visage examinées met nettement en évidence comment la préparation de liposomes d'eau déminéralisée et de collagène n'était pas en mesure de modifier de manière statistiquement significative les valeurs cornéométriques (voir figure 7A) dans la casuistique étudiée.

Par contre, le traitement par des liposomes d'eau oligominérale "Sorgente Panna" a donné lieu à des valeurs de moyennes globales rapportées dans la figure 7B. On soulignera que, bien que les deux groupes étudiés comprennent des sujets d'âges différents, la différence entre les valeurs moyennes plus ou moins $\sigma$ (écart-type) au temps $T = 0$ entre les deux groupes n'est pas statistiquement significative ($t = 1,347225933$ ; degrés de liberté : 10), de sorte que les deux groupes doivent être considérés comme statistiquement homogènes.

L'analyse statistique des valeurs moyennes plus ou moins $\sigma$ à la trentième minute met en évidence une différence statistiquement significative ($P < 0,02$) en faveur du groupe traité avec les liposomes d'eau oligominérale "Sorgente Panna" ($t = 5,318190528$ ; degrés de liberté : 10).

De manière analogue,à la soixantième minute, la différence entre les valeurs moyennes obtenues avec les liposomes d'eau déminéralisée de l'exemple 2 et d'eau oligominérale "Sorgente Panna" de l'exemple 1 s'est révélée statistiquement significative (P < 0,02 ; t = 2,36832777, degrés de liberté : 10).

TABLEAU I

Détermination du gradient d'hydratation de la couche cornée par cornéométrie : front, régions temporo-zygomatiques droite et gauche, joues droite et gauche, menton et moyenne des valeurs de tout le visage dans les conditions initiales et 30 et 60 min après l'application liposomique d'eau déminéralisée plus collagène.

| T, min | F | T.Z.dr. | T.Z.g | J.dr. | J.g. | M | Cornéométrie globale |
|---|---|---|---|---|---|---|---|
| 0 | 72,2 ± 28,3 | 83,0 ± 16,5 | 98,7 ± 2,5 | 90,2 ± 6,7 | 97,2 ± 7,6 | 96,2 ± 6,9 | 89,5 ± 10,3 |
| 30 | 77,2 ± 12,4 | 91,7 ± 5,3 | 91,2 ± 9,4 | 83,5 ± 8,1 | 88,2 ± 5,6 | 78,5 ± 19,8 | 85,05 ± 6,3 |
| 60 | 69,2 ± 23,9 | 84,7 ± 9,0 | 89,5 ± 16 | 79,5 ± 7,7 | 81,5 ± 8,8 | 81,7 ± 8,5 | 81,01 ± 6,7 |

T = temps, F = front, T.Z.dr. = région temporo-zygomatique droite, T.Z.g. = région temporo-zygomatique gauche, J.dr. = joue droite, J.g. = joue gauche, M = menton, cornéométrie globale = moyenne des valeurs cornéométriques des différentes régions cutanées du visage.

## TABLEAU II

Détermination du gradient d'hydratation de la couche cornée par cornéométrie : front, régions temporo-zygomatiques droite (T.Z.dr.) et gauche (T.Z.g.), joues droite (J.dr.) et gauche (J.g.), menton (M) et moyenne des valeurs de tout le visage dans les conditions initiales et 30 et 60 min après l'application des liposomes d'eau oligominérale "Sorgente Panna" plus GAG.

| T, min | F | T.Z.dr. | T.Z.g. | J.dr. | J.g. | M | Cornéométrie globale |
|---|---|---|---|---|---|---|---|
| 0 | $93,3 \pm 9,71$ | $95,3 \pm 10,99$ | $94,8 \pm 12,00$ | $93,9 \pm 13,42$ | $94,5 \pm 14,16$ | $100,9 \pm 6,78$ | $95,4 \pm 2,8$ |
| 30 | $101,9 \pm 9,18$ | $100,7 \pm 11,29$ | $99,7 \pm 11,23$ | $99,4 \pm 13,59$ | $99,6 \pm 10,51$ | $108,6 \pm 4,69$ | $101,6 \pm 3,5$ |
| 60 | $100,5 \pm 9,64$ | $96,9 \pm 10,39$ | $98,7 \pm 9,76$ | $98,8 \pm 8,84$ | $98,3 \pm 11,84$ | $108,4 \pm 6,73$ | $100,2 \pm 4,1$ |

EP 0 274 363 B1

**Revendications**

1. Procédé pour un traitement hydratant de la peau, utilisable en particulier dans le domaine cosmétique, caractérisé en ce qu'il comprend l'administration locale à la peau de liposomes qui contiennent des précurseurs biochimiques immédiats des glucosaminoglycannes désulfurés et eau minérale.

2. Procédé selon la revendication 1, caractérisé en ce que ladite eau minérale est une oligominérale.

3. Procédé selon la revendication 2, caractérisé en ce que ladite eau oligominérale est l'eau oligominérale "Sorgente Panna".

4. Procédé selon la revendication 1, caractérisé en ce que l'administration d'eau minérale Par voie liposomique est effectuée par distribution de cette eau à l'état nébulisé ou atomisé par de petites bombes dans lesquelles on a préalablement conditionné l'eau minérale liposomique et un agent propulseur approprié.

5. Produit pour un traitement hydratant efficace de la peau, utilisé en particulier, dans le domaine cosmétique, caractérisé en ce qu'il comprend des liposomes qui contiennent eau oligominérale et précurseurs biochimiques immédiats des glucosaminoglycannes désulfurés.

6. Produit selon la revendication 5, caractérisé en ce que ladite eau oligominérale est l'eau oligominérale "Sorgente Panna".

**Claims**

1. A method for a moisturizing treatment of the skin, useful particularly for cosmetics applications, characterized in that it comprises the topical administration to the skin of liposomes which contain immediate biochemical precursors of the asulfurate glucosaminoglycanes and mineral water.

2. A method according to Claim 1, characterized in that said mineral water is oligomineral water.

3. A method according to Claim 2, characterized in that said oligomineral water is "Sorgente Panna" oligomineral water.

4. A method according to Claim 1, characterized in that the administering of mineral water by way of liposomes is carried out by delivering said water in a nebulized or atomized state from small bottles wherein the liposomic water and a suitable propellant have been previously packed.

5. A product for an effective moisturizing treatment of the skin, particularly in cosmetics applications, characterized in that it comprises liposomes which contain oligomineral water and immediate biochemical precursors of the asulfurate glucosaminoglycanes.

6. A product according to Claim 5, characterized in that said oligomineral water is "Sorgente Panna" oligomineral water.

**Patentansprüche**

1. Verfahren zur Feuchthaltung der Haut, insbesondere zur Verwendung in der Kosmetik, gekennzeichnet durch die lokale Verabreichung von Liposomen an die Haut, welche direkte biochemische Zwischenstoffe von entsulfurierten Glucosamininoglykanen und Mineralwasser enthalten.

2. Verfahren nach Anspruch 1, dadurch gekennzeichnet, daß das genannte Mineralwasser ein oligomineralisches Wasser ist.

3. Verfahren nach Anspruch 2, dadurch gekennzeichnet, daß das genannte oligomineralische Wasser das oligomineralische Wasser "Sorgente Panna" ist.

4. Verfahren nach Anspruch 1, dadurch gekennzeichnet, daß die Verabreichung von Mineralwasser auf liposomischem Weg durch Verteilung dieses Wassers in zerstäubtem oder atomisiertem Zustand in kleinen Bomben vorgenommen wird, in welche das liposomische Mineralwasser und ein geeignetes Treibmittel zuvor abgepackt wurden.

5. Mittel zur Feuchthaltung der Haut, insbesondere zur Verwendung in der Kosmetik, dadurch gekennzeichnet, daß es aus Liposomen besteht, welche oligomineralisches Wasser und direkte biochemische Zwischenstoffe von entsulfurierten Glucosaminoglykanen enthalten.

6. Mittel nach Anspruch 5, dadurch gekennzeichnet, daß das genannte oligomineralische Wasser das oligomineralische Wasser "Sorgente Panna" ist.

Fig-1A

Fig-1B

Fig-2A

Fig-2B

Fig-3A

Fig-3B

Fig - 4A

Fig - 4B

Fig - 5A

Fig - 5B

Fig - 6A

Fig - 6B

Fig-7A

Fig-7B